# EUROPÄISCHE PATENTSCHRIFT

(11) **EP 1 440 756 B1**
(45) Veröffentlichungstag und Bekanntmachung des Hinweises auf die Patenterteilung: **18.05.2005**
(21) Anmeldenummer: 02028241.4
(22) Anmeldetag: 16.12.2002
(51) Int. Cl.: B23D 29/00, B23D 29/02, A61F 15/02

(54) **Motorische Schneidevorrichtung**
Motorised cutting device
Dispositif de coupe motorisé

(43) Veröffentlichungstag der Anmeldung: 28.07.2004
(73) Patentinhaber: Kratzmaier Konstruktionen GmbH, 82547 Eurasburg (DE)
(72) Erfinder: Kratzmaier, Erich, 82547 Eurasburg (DE)
(74) Vertreter: Szynka, Dirk, Dr.

(56) Entgegenhaltungen:
- DE-C- 719 969
- US-A- 3 025 599
- US-A- 4 682 416
- US-A- 5 956 992

## Beschreibung

Diese Erfindung bezieht sich auf eine motorisch angetriebene Schneidevorrichtung zum Schneiden flacher Gegenstände. Die Vorrichtung soll als Handgerät eingesetzt werden, also als Gesamtvorrichtung in der Hand oder mit den Händen zu halten sein. Die Erfindung richtet sich bevorzugt auf Vorrichtungen, die ausgelegt sind zum Aufschneiden von einen menschlichen Körper umgebenden flachen Gegenständen, insbesondere Hartverbänden, Schutzanzügen wie Motorradanzügen und dergleichen. Sie richtet sich aber auch ganz allgemein auf mit der Hand haltbare Schneidvorrichtungen zum Aufschneiden flacher Gegenstände anderer Art.

Speziell beim Aufschneiden von Verbänden und insbesondere Hartverbänden ergeben sich größere Schwierigkeiten bei der Verwendung rein handbetriebener Scheren und anderer Werkzeuge. Gewöhnliche Verbände sind bereits relativ schwer mit einer Schere aufzuschneiden, wobei durch die Ausübung körperlicher Kraft ein gewisses Verletzungsrisiko für den Patienten besteht. Beim Aufschneiden von Hartverbänden aus Gips oder Kunststoffmaterialien treten diese Schwierigkeiten in besonders ausgeprägter Weise auf.

Ähnliche Probleme ergeben sich aber auch beispielsweise beim Öffnen eines Motorradschutzanzuges bei der ersten Hilfe an einem Unfallort. Dort muss der Rettungssanitäter oder Arzt möglichst schnell mit einfachen Mitteln Verletzungen feststellen können und Körperstellen zugänglich machen. Der Motorradschutzanzug kann mit Rücksicht auf eventuelle Knochenverletzungen, insbesondere Wirbelbrüche, nicht mehr konventionell ausgezogen werden. In diesen Fällen wäre es für die Effizienz der Diagnose und Versorgung sehr vorteilhaft, mit einfachen Mitteln schnell einen Schutzanzug aufschneiden zu können. Analog gilt dies auch für andere Situationen, in denen beispielsweise bei einem Unfall ein Schutzanzug einer verletzten Person aufgeschnitten werden muss.

Der Erfindung liegt somit das technische Problem zugrunde, eine vorteilhafte Schneidevorrichtung für flache Gegenstände anzugeben.

Die DE 719 969 zeigt eine Vorrichtung zum Schneiden flacher Gegenstände nach dem Oberbegriff des Anspruchs 1. Ferner zeigt die US 4 682 416 eine von einer Handbohrmaschine angetriebene scherenartige Vorrichtung zum Schneiden von dünnen Blechen. Gleiches gilt für die US 3 025 599.

Die Erfindung richtet sich zum einen auf eine Vorrichtung zum Schneiden flacher Gegenstände, die mit den Händen gehalten werden kann und eine Antriebsmotoreinheit und eine Schneidwerkzeugeinheit aufweist, die zumindest ein bewegbares und durch die Antriebsmotoreinheit antreibbares Schneidwerkzeug und zumindest ein weiteres Schneidwerkzeug aufweist, wobei das bewegbare Schneidwerkzeug relativ zu dem weiteren Schneidwerkzeug bewegbar ist und die Bewegung einer Schneidkante des bewegbaren Schneidwerkzeugs einer geschlossenen Bahn entspricht, die eine endliche Fläche umläuft, dadurch gekennzeichnet, dass das zumindest eine bewegbare Schneidwerkzeug an einer von seiner Schneidkante entfernten Lagerachse drehbar und verschiebbar gelagert ist und an einem seiner Schneidkante näheren Exzenter gelagert und von diesem antreibbar ist.

Daneben richtet sich die Erfindung auch auf ein entsprechendes Verfahren, bei dem die Vorrichtung nach Anspruch 1 in der Hand gehalten und mit der Schneidwerkzeugeinheit an der flachen Vorrichtung geführt wird, wobei die Schneidwerkzeugeinheit die flache Vorrichtung durch die Antriebsmotoreinheit angetrieben aufschneidet.

Vorteilhafte Ausgestaltungen sind in den abhängigen Ansprüchen angegeben und werden in der folgenden Beschreibung im Einzelnen erläutert, wobei die jeweiligen Merkmale sowohl im Hinblick auf den Verfahrensaspekt als auch auf den Vorrichtungsaspekt der Erfindung zu verstehen sind.

Die Vorrichtung verfügt also neben der Antriebsmotoreinheit über eine Schneidwerkzeugeinheit mit zumindest zwei Schneidwerkzeugen. Davon ist zumindest eines durch die Antriebsmotoreinheit antreibbar und bewegt sich in angetriebenem Zustand relativ zu dem anderen Schneidwerkzeug. Es kann sich also beispielsweise um ein bewegbares und ein relativ zu der Antriebsmotoreinheit feststehendes Schneidwerkzeug oder auch um zwei relativ zueinander und zu der Antriebsmotoreinheit bewegbare Schneidwerkzeuge handeln. Insbesondere kann es sich bei diesen Schneidwerkzeugen um messerähnliche Werkzeuge handeln. Der Schneidvorgang selbst kann dabei durch die Schärfe einer Schneidkante im Sinne eines Messers oder auch durch ein scherenähnliches Schneiden zwischen zwei Kanten der zumindest zwei Schneidwerkzeuge erfolgen. Es sind natürlich auch Mischfälle denkbar. Bei dem Schneidvorgang kommt es zu Relativbewegungen zwischen dem flachen Gegenstand und den Schneidwerkzeugen senkrecht zu der Ebene des flachen Gegenstands und auch in dieser Ebene. Für die Erfindung ist die Form der Bewegung des bewegbaren Schneidwerkzeugs gegenüber dem anderen Schneidwerkzeug von Bedeutung. Diese Bewegung soll nicht entlang ein und derselben Linie hin- und herlaufen sondern eine geschlossene Bahn beschreiben, die eine endliche Fläche umschließt. Denkt man sich also einen Punkt der Schneidkante des bewegbaren Schneidwerkzeugs und verfolgt diesen während der Bewegung relativ zu dem anderen Schneidwerkzeug, so ergibt sich eine letztlich geschlossene Linie. Diese soll eine Fläche einschließen, also nicht nur einer linienhaften Hin- und Herbewegung entsprechen. Diese Fläche kann rundlich sein, muss jedoch nicht kreisrund sein. Beispielsweise kann sie elliptisch oder in anderer Form flächig rundlich sein.

Damit ergibt sich nach den Erfahrungen des Erfinders eine günstige Kopplung verschiedener Bewegungskomponenten zwischen den Schneidwerkzeugen und dem zu schneidenden flachen Gegenstand. Insbesondere sind dabei senkrecht zu der Ebene des flachen Gegenstands und in dieser Ebene liegende Bewegungskomponenten vertreten, die ein günstiges Schneidergebnis erzeugen. Die vorstehenden Ausführungen gelten dabei sowohl für Relativbewegungen zwischen einem bewegbaren und einem weiteren feststehenden Schneidwerkzeug also auch für zwei bewegbare Schneidwerkzeuge.

Die Bewegung in der Vorrichtung wird dadurch erzeugt, dass das oder die Schneidwerkzeug/e einerseits, und zwar in einem von der Schneidkante entfernten Bereich, drehbar und verschiebbar entlang einem Führungsstift oder allgemein gesprochen einer Lagerachse gelagert ist/sind und andererseits an einer der Schneidkante deutlich näheren Stelle durch einen Exzenter angetrieben ist/sind. Durch die Kopplung zwischen der Lagerung an dem Exzenter und der entfernten drehbaren und verschiebbaren Lagerung ergeben sich dann an der Schneidkante die beschriebenen Bewegungsformen. Es wird auf das Ausführungsbeispiel verwiesen.

Bei einer Ausgestaltung der Vorrichtung ist die Antriebsmotoreinheit mit der Schneidwerkzeugeinheit in einer lösbaren Weise verbunden, die Schneidwerkzeugeinheit also von der Restvorrichtung abnehmbar. Der Begriff "abnehmbar" meint dabei natürlich keine vollständige Zerlegung der Vorrichtung und ein nachfolgendes Trennen der jeweiligen Teile, sondern ein Abnehmen ohne oder mit nur einfachem Werkzeug ohne Zerlegung der getrennten Einheiten in jeweilige Einzelteile und vorzugsweise ohne das jeweilige Gehäuse öffnen zu müssen. Gemeint ist beispielsweise das Lösen einer oder weniger Befestigungsschrauben und nachfolgende Abziehen. Hierdurch kann die Schneidwerkzeugeinheit leicht gereinigt oder bei Verwendung im medizinischen Bereich auch sterilisiert werden. Insbesondere muss mit Reinigungs- und Sterilisiervorgängen nicht auf die Antriebsmotoreinheit Rücksicht genommen werden, die beispielsweise hinsichtlich einer thermischen Sterilisierung deutlich empfindlicher sein kann. Es können auch mehrere Schneidwerkzeugmodule vorgehalten werden, so dass für unterschiedliche Aufgaben abweichend voneinander ausgestaltete Varianten leicht ausgetauscht werden können oder ein stumpfes oder verschmutztes Schneidwerkzeugmodul durch ein anderes ersetzt werden kann.

Vorzugsweise ist nicht nur die Schneidwerkzeugeinheit von der Antriebsmotoreinheit in der erwähnten Weise abnehmbar, sondern ist auch eine zwischen Schneidwerkzeugeinheit und Antriebsmotoreinheit vorgesehene Getriebeeinheit einerseits von der Schneidwerkzeugeinheit und andererseits von der Antriebsmotoreinheit abnehmbar. Damit ergeben sich zum einen die beschriebenen Vorteile auch für die Getriebeeinheit. Zum Zweiten können unterschiedliche Getriebe mit unterschiedlichen Übersetzungsverhältnissen oder unterschiedlichen Winkeln oder auch unterschiedlichen Längen zwischen Antriebsmotoreinheit und Schneidwerkzeugeinheit eingesetzt werden.

Eine weitere Ausgestaltung der Vorrichtung sieht ein Kugellager in der Schneidwerkzeugeinheit vor, mit dem das zumindest eine bewegbare Schneidwerkzeug gelagert ist. Es hat sich erwiesen, dass im harten Einsatz einer erfindungsgemäßen Vorrichtung durch Reibungsverluste in der Schneidwerkzeugeinheit erhöhte Temperaturen auftreten können, die entweder wegen Verletzungsgefahr eines Patienten oder Unfallopfers oder mit Rücksicht auf das zu schneidende Material vermieden werden sollen. Dabei hat es sich als sehr vorteilhaft erwiesen, die geschilderte Bewegung des bewegbaren Schneidwerkzeugs durch Verwendung zumindest eines Kugellagers reibungsfreier zu gestalten. Das Kugellager findet dabei zur Lagerung des Schneidwerkzeugs an dem Exzenter Verwendung. Zur Veranschaulichung wird auf das Ausführungsbeispiel verwiesen.

Eine weitere Ausgestaltung der Vorrichtung betrifft eine vorteilhafte Temperaturerfassung in der Schneidwerkzeugeinheit. Dabei kann zusätzlich zu dem erwähnten Kugellager oder alternativ dazu durch Messen einer typischen Temperatur in der Nähe der Schneidwerkzeuge und entsprechende Reaktion der Vorrichtung, also etwa eine Warnanzeige oder einen Sicherheitsausschaltvorgang, eine Gefährdung eines Menschen oder des zu schneidenden Materials oder auch der Vorrichtung selbst vermieden werden. Hier eignen sich insbesondere Thermoelemente.

Die Schneidwerkzeuge selbst sind vorzugsweise im Wesentlichen plan und gleiten in ihrer Relativbewegung flächig aneinander entlang. Sie können eine konvexe Schneidkante aufweisen, die entweder eine Scherwirkung oder messerähnliche Wirkung auf das zu schneidende Material erlaubt. Die Schneidkante kann insbesondere auch gezahnt sein, um den zu schneidenden Gegenstand besonders gut zu greifen. Die Zahnung kann dabei vor allem auch an nur einer Schneidkante, etwa der des bewegbaren Schneidwerkzeugs, vorgesehen sein.

Zwischen einer Schneidkante eines bewegbaren Schneidwerkzeugs und dem zu schneidenden flachen Gegenstand kann es dabei gemäß einer weiteren vorteilhaften Ausgestaltung der Erfindung zu einer deutlichen Reibung kommen, insbesondere infolge der erwähnten Zahnung, so dass sich die Vorrichtung mit ihren Schneidwerkzeugen infolge der eine Komponente in der Ebene des flachen Gegenstands aufweisenden Bewegungsteile entlang einer Schnittlinie durch den Gegenstand fortbewegt. Die erfindungsgemäße Schneidwerkzeugbewegung kann also neben verbesserten Schneidresultaten auch eine Vereinfachung der Führung der Vorrichtung durch eine Bedienungsperson bieten, die nicht mehr allein durch Körperkraft einen ausreichenden Druck zum Entlangschieben der Schneidwerkzeugeinheit entlang dem Verband oder dem Anzug oder einem anderen Gegenstand aufbringen muss.

Bei einer bevorzugten Ausgestaltung verfügt die erfindungsgemäße Schneidwerkzeugeinheit sowohl über zwei bewegbare Schneidwerkzeuge als auch über ein weiteres feststehendes Schneidwerkzeug, das zwischen diesen bewegbaren Schneidwerkzeugen angeordnet ist. Dadurch ergibt sich beidseits des feststehenden Schneidwerkzeugs ein Schneidevorgang, so dass die Vorrichtung besonders effizient arbeitet. Bei versetzten Bewegungen der beiden bewegbaren Schneidwerkzeuge kann, wenn gewünscht, ein Massenausgleich erzielt werden. Schließlich ist es möglich, mit den beiden bewegbaren Schneidwerkzeugen beidseits des eine gewisse Breite aufweisenden feststehenden Schneidwerkzeugs nicht nur eine Linie in den flachen Gegenstand sondern einen Streifen aus dem flachen Gegenstand heraus zu schneiden. Dies kann das nachfolgende Öffnen eines Hartverbands oder Schutzanzugs erleichtern.

Die Antriebsmotoreinheit erlaubt vorzugsweise eine stufenlose Geschwindigkeitseinstellung der Motorbewegung und damit der Schneidwerkzeugbewegungen. Insbesondere kann die Antriebsmotoreinheit einen Taster zum Einschalten und, infolge Loslassens, Ausschalten der Antriebsmotoreinheit aufweisen, wobei die Intensität des Drucks auf den Taster oder die Strecke, um die der Taster in eine Führung hineingeschoben wird, zur Einstellung der Antriebsgeschwindigkeit dienen kann.

Eine weitere Ausgestaltung der Vorrichtung bezieht sich darauf, die Antriebsmotoreinheit in einer ohnehin im Markt erhältlichen Form zu wählen, und zwar insbesondere als gewöhnliche Handbohrmaschine oder als elektrischen Handschrauber mit Akkuantrieb oder Netzkabel. Beispielsweise kann ein solches Gerät ohne das entsprechende Futter zur Befestigung von Bohrern oder Schrauberbits Verwendung finden. Auf die Abtriebswelle, die gewöhnlich ein entsprechendes Gewinde aufweist, kann dann ein Getriebe der erfindungsgemäßen Vorrichtung aufgesetzt werden. Dabei kann beispielsweise zuvor auf die Abtriebswelle ein Zahnrad aufgebracht worden sein. Die Erfindung bezieht sich also auch auf eine entsprechend den vorstehenden Erläuterungen ausgestaltete Schneidevorrichtung, der jedoch die Antriebsmotoreinheit fehlt und die vielmehr zur Ankupplung an eine solche konventionelle Antriebsmotoreinheit ausgelegt ist.

Die Vorrichtung kann sich also insbesondere aus drei Modulen zusammensetzen, zum einen einer modular abnehmbaren Schneidwerkzeugeinheit, zum Zweiten einer modular zwischengeschalteten Getriebeeinheit und zum Dritten einer konventionellen Bohr- oder Schraubmaschine als Antriebsmotoreinheit.

Im Folgenden wird die Erfindung anhand eines Ausführungsbeispiels näher erläutert, das in den beiliegenden Figuren dargestellt ist. Dabei offenbarte Einzelmerkmale können auch in anderen Kombinationen erfindungswesentlich sein.
- Fig. 1: zeigt eine perspektivische Darstellung einer erfindungsgemäßen Vorrichtung.
- Fig. 2: zeigt die Vorrichtung aus Fig. 1, jedoch mit abgenommener Schneidwerkzeugeinheit.
- Fig. 3: zeigt eine Explosionsdarstellung der Schneidwerkzeugeinheit aus Fig. 2.
- Fig. 4: zeigt eine Ansicht der Vorrichtung aus den Fig. 1 und 2 in einer abweichenden Perspektive und bei abgenommener Schneidwerkzeugeinheit, wobei die Getriebeeinheit teilweise zerlegt dargestellt ist.

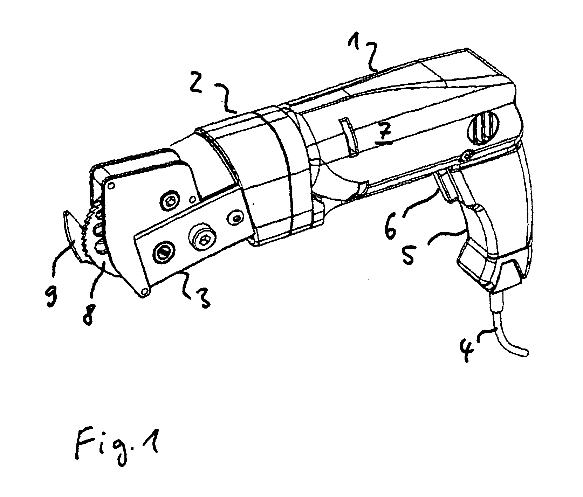

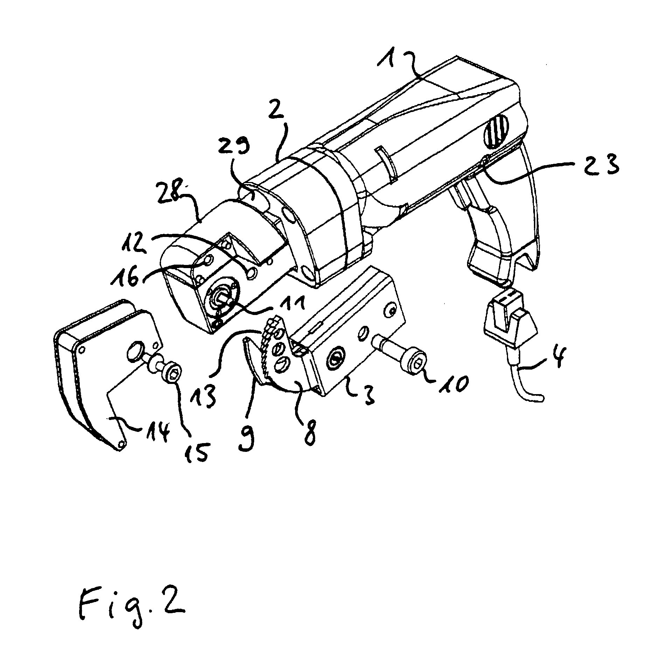

In Fig. 1 erkennt man eine erfindungsgemäße Schneidevorrichtung mit einer Antriebsmotoreinheit 1, einer Getriebeeinheit 2 und einer Schneidwerkzeugeinheit 3. Bei der Antriebsmotoreinheit 1 handelt es sich um eine konventionelle elektrische Bohrmaschine mit einem Stromversorgungskabel 4, einem Bedienungshandgriff 5, einem Taster 6 zum Einschalten und zur Geschwindigkeitssteuerung und schließlich mit einem in Fig. 1 nicht sichtbaren, jedoch in dem mit "7" bezeichneten Gehäuseteil angeordneten elektrischen Motor. An die in Fig. 1 links vorne liegende Seite der Antriebsmotoreinheit 1 ist eine Getriebeeinheit 2 angesetzt, die im Wesentlichen eine Untersetzung zur Erhöhung des Drehmoments und Verringerung der Drehgeschwindigkeit und zum Umlenken der Rotationsrichtung um 90° enthält. Die Abtriebswelle der Antriebsmotoreinheit 1 (vgl. 11 in Fig. 2) liegt nämlich, wie bei solchen Bohrmaschinen üblich, längs der Längsachse der in Fig. 1 gezeigten Gesamtvorrichtung. Die Drehachse der in Fig. 1 nicht gezeigten Abtriebswelle der Getriebeeinheit 2 (vgl. Fig. 2) liegt jedoch senkrecht dazu und dabei waagrecht (bei nach unten weisendem Handgriff 5).

Die an die Getriebeeinheit 2 angekoppelte Schneidwerkzeugeinheit 3 weist drei Schneidwerkzeuge auf, von denen in Fig 1 ein bewegbares Schneidwerkzeug 8 und ein feststehendes Schneidwerkzeug 9 zu sehen ist.

Fig.2 zeigt die Vorrichtung aus Fig.1, wobei die Schneidwerkzeugeinheit 3 nach Lösen einer Imbusbefestigungsschraube 10 von der Getriebeeinheit 2 abgenommen worden ist. Ferner erkennt man die bereits erwähnte Abtriebswelle der Getriebeeinheit 2, die mit 11 bezeichnet ist, und ein daneben liegendes Gewindesackloch 12 für die Befestigungsschraube 10. Die Kopplung zwischen der Schneidwerkzeugeinheit 3 und der Getriebeeinheit 2 ist ferner durch hier nicht näher eingezeichnete Verdrehsicherungsstifte und -ausnehmungen stabilisiert, so dass die Abtriebswelle 11 und das dazu passende Kupplungsstück der Schneidwerkzeugeinheit 3 beim Festziehen der Schraube 10 nicht belastet werden.

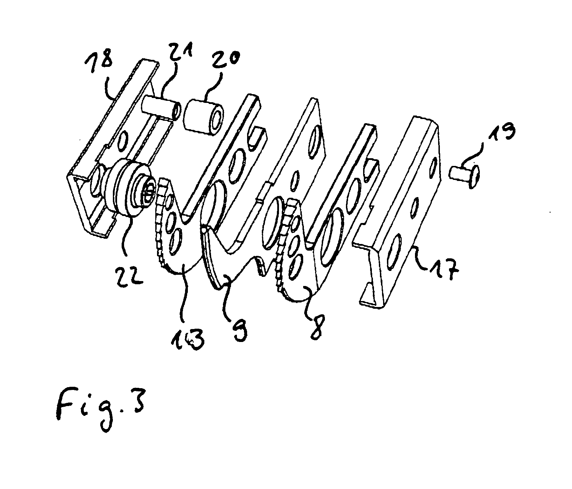

Fig. 2 zeigt außerdem ein zweites bewegbares Schneidwerkzeug 13, auf das noch näher anhand Fig. 3 eingegangen wird. Die Figuren zeigen ferner, dass die bewegbaren Schneidwerkzeuge 8 und 13 zu der der Antriebsmotoreinheit 1 entgegengesetzten Seite ungefähr halbkreisförmig konvexe Schneidkanten mit Zahnungen aufweisen, wohingegen das feststehende Schneidwerkzeug 9 hier eine weniger gekrümmte, jedoch etwas konvexe Gleitkante ohne Zahnung aufweist.

Schließlich zeigt Fig. 2 eine Schutzhaube 14, die mit einer Befestigungsschraube 15 in einem Gewindesackloch 16 der Getriebeeinheit 2 angebracht werden kann. Diese Schutzhaube 14 ist für die Funktion der Erfindung nicht notwendig, verringert aber die Gefahr unbeabsichtigter Verletzungen, insbesondere an den Fingern der Bedienungsperson. Dazu deckt sie im Betrieb die Schneidwerkzeuge 8, 9 und 13 weitgehend ab und wird von einem zu schneidenden flachen Gegenstand beim Heranführen der Schneidwerkzeuge 8, 9, 13 im Wesentlichen auf die Antriebsmotoreinheit 1 zu hochgeschoben. Dazu ist der Teil 28 der Getriebeeinheit 2, an dem die Schutzhaube 14 befestigt werden kann, entlang der Längsachse der Vorrichtung und mit Hilfe der Führung 29 verschiebbar.

Im Unterschied zu Fig. 1 ist in Fig. 2 im Übrigen das Kabel 4 von der Antriebsmotoreinheit 1 abgezogen.

Fig. 3 zeigt nur die Schneidwerkzeugeinheit 3 aus den Fig. 1 und 2, und zwar in einer Explosionsdarstellung. Diese zeigt neben den bereits erwähnten Schneidwerkzeugen 8, 9 und13 im Wesentlichen zwei Gehäuseschalenteile 17 und 18. Eine Befestigungsschraube 19 hält diese beiden Gehäuseschalenteile 17 und 18 im montierten Zustand zusammen und durchsetzt dabei nach einer zu der Antriebsmotoreinheit 1 orientierten Seite hin offene Ausnehmungen an den bewegbaren Schneidwerkzeugen 8 und 13 sowie eine Bohrung in dem feststehenden Schneidwerkzeug 9. Zwischen den Ausnehmungen und der Bohrung einerseits und einem zu der Gehäuseschalenhälfte 18 gehörenden Aufnahmezapfen 21 für die Befestigungsschraube 19 ist eine Gleithülse 20 vorgesehen. Infolge der einseitig offenen Ausnehmungen sind die bewegbaren Schneidwerkzeuge 8 und 13 also auf der Gleithülse 20 gleitend drehbar und verschiebbar gelagert.

Die Schneidwerkzeuge 8, 9 und 13 weisen ferner Öffnungen für die in Fig. 2 eingezeichnete Befestigungsschraube 10 auf, die im Fall der bewegbaren Schneidwerkzeuge 8 und 13 ein die Bewegung derselben berücksichtigendes Spiel haben. Schließlich weisen die Schneidwerkzeuge 8, 9 und 13 jeweils eine relativ große kreisrunde Bohrung auf, die im montierten Zustand von einem Exzenter 22 durchsetzt ist. Der Exzenter 22 weist in den Bereichen seines größten Durchmessers zwei axial nebeneinander liegende Kugellager und in den beiden dem gegenüber axial außen liegenden Bereichen mit kleinerem Durchmesser jeweils ein weiteres Kugellager auf. Die beiden äußeren kleineren Kugellager durchsetzen im montierten Zustand die in Fig. 3 erkennbaren zugehörigen Bohrungen in den Gehäuseschalenteilen 17 und 18, so dass der Exzenter 22 gegenüber diesen Gehäuseschalenteilen 17 und 18 drehbar ist, ohne eine Gleitreibung zu erzeugen. Entsprechendes gilt für die größeren axial inneren Kugellager, die die Öffnungen in den bewegbaren Schneidwerkzeugen 8 und 13 durchsetzen. Sie lassen zwischen sich einen in Fig. 3 angedeuteten Spalt für das feststehende Schneidwerkzeug 9. Wenn also eine innerhalb der Kugellager des Exzenter 22 liegende Antriebsachse durch die in Fig. 2 erkennbare Abtriebswelle 11 der Getriebeeinheit 2 drehend angetrieben wird, ergibt sich zwischen dieser Antriebsachse und den Gehäuseschalenteilen 17 und 18 sowie dem bewegbaren Schneidwerkzeugen 8 und 13 jeweils eine Rollreibung in den entsprechenden Kugellagern und ist das feststehende Schneidwerkzeug 9 von dem Exzenter 22 frei.

Die exzentrische Bewegung des Exzenters 22 betrifft nur die beiden axial inneren größeren Kugellager und überträgt sich damit in oval kreisende Bewegungen der Schneidkanten der bewegbaren Schneidwerkzeuge 8 und 13, die dabei um die Gleithülse 20 hin und her verdreht und an ihr entlang etwas verschoben werden. Die Exzentrizität der beiden größeren Kugellager des Exzenter 22 ist dabei um 180° phasenversetzt, so dass die beiden Schneidwerkzeuge 8 und 13 entsprechend phasenversetzt bewegt werden.

Im Betrieb wird die Vorrichtung mit dem feststehenden Schneidwerkzeug 9 (vgl. Fig. 1) an einem Rand beispielsweise eines Hartverbands unter diesen Hartverband gebracht und durch Antippen des Tasters 6 eine Antriebsbewegung der bewegbaren Schneidwerkzeuge 8 und 13 erzeugt. Diese bewegen sich im Sinne der Fig. 1 und 2 gegen den Uhrzeigersinn und werden bei dieser kreisenden Bewegung zum einen immer wieder auf den Hartverband herabgedrückt, wobei dieser durch das Schneidwerkzeug 9 gehalten ist. Zum anderen werden sie nach der Hälfte dieser Abwärtsbewegung (d. h. in Fig. 1 nach links) sozusagen nach rückwärts bewegt (d. h. in Fig. 1 nach unten), so dass sich eine Vorwärtsbewegung der Vorrichtung gegenüber dem Hartverband ergibt. Im Gebrauch steht also die Längsachse der Vorrichtung aus Fig. 1 (die Rotationsachse der Abtriebswelle der Antriebsmotoreinheit 1) ungefähr senkrecht auf dem zu schneidenden flachen Gegenstand, insbesondere Hartverband. Die beiden bewegbaren Schneidwerkzeuge 8 und 13 laufen sozusagen auf dem Verband entlang und schneiden ihn dabei bei den "abwärtstretenden" Bewegungen auf, wobei das feststehende Schneidwerkzeug 9 als Gegenlager verwendet wird. In diesem Fall handelt es sich dabei teils um einen sägeähnlichen Vorgang infolge der in den Figuren erkennbaren Zahnung der bewegbaren Schneidwerkzeuge 8 und 13 und zum anderen Teil um ein Abscheren zwischen den bewegbaren Schneidwerkzeugen 8 und 13 und dem feststehenden Schneidwerkzeug 9. Dabei müssen die bewegbaren Schneidwerkzeuge 8 und 13 mit den gezahnten Schneidkanten nicht über den Abschluss des feststehenden Schneidwerkzeugs 9 hinaus bewegt werden, so dass sich, wenn dieses beispielsweise auf der Haut eines Patienten oder Unfallopfers entlang gleitet, keine Verletzungsgefahr ergibt.

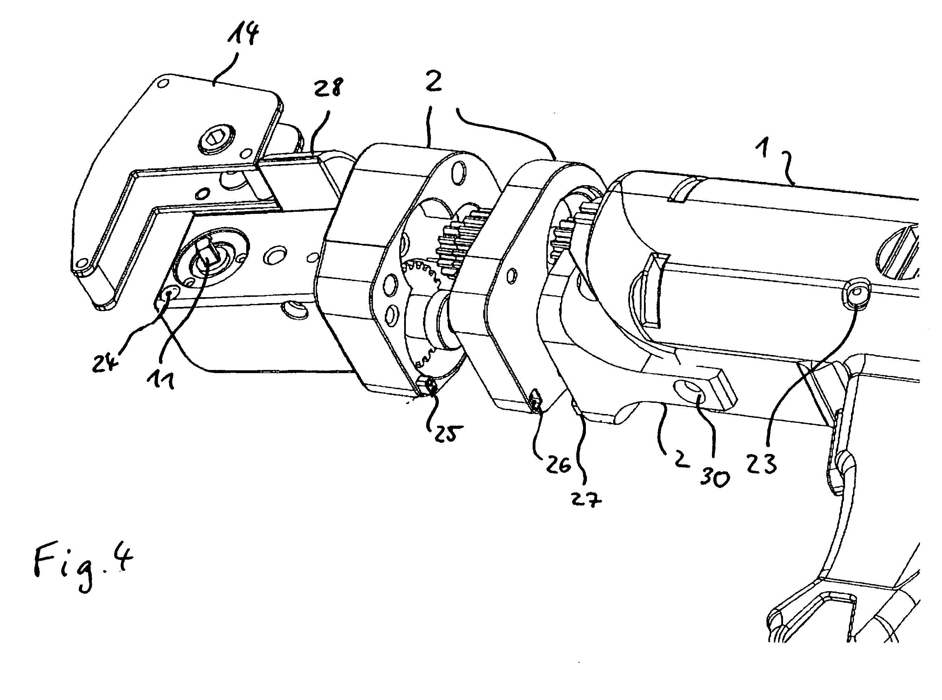

An der der Schneidwerkzeugeinheit 3 zugewandten Seite weist die Getriebeeinheit 2 ferner einen in Fig. 4 mit 24 eingezeichneten Thermoelementfühler auf, der über eine elektrische Kupplung zwischen der Getriebeeinheit 2 und der Antriebsmotoreinheit 1 ein Spannungssignal überträgt und mit einer LED-Anzeige 23 gekoppelt ist. Bei Überhitzung der Schneidwerkzeugeinheit 3 leuchtet daher die LED-Anzeige 23 auf und warnt den Benutzer rechtzeitig.

Infolge der Verwendung der Kugellager des Exzenters 22 sind die Reibungsverluste in der Schneidwerkzeugeinheit 3 ohnehin begrenzt, so dass die Überhitzung im Vergleich zu Gleitreibungsverlusten später auftritt. Die zwischen den bewegbaren Schneidwerkzeugen 8 und 13 und der Gleithülse 20 erzeugte Gleitreibungswärme hat sich als vergleichsweise unwesentlich erwiesen. Natürlich könnten auch hier Kugellager eingesetzt werden. Bei einem seitlichen Versatz zwischen den neben den einseitig offenen Ausnehmungen der Schneidwerkzeuge 8 und 13 stehenden Stegen ("U-Schenkel") könnte jedem dieser Stege ein Kugellager zugeordnet werden, so dass auch hier nur Rollreibung auftreten würde.

Fig. 4 zeigt die Vorrichtung aus den Fig. 1 und 2 mit Antriebsmotoreinheit 1, Getriebeeinheit 2 und Abdeckung 14, jedoch ohne Schneidwerkzeugeinheit 3. Zunächst erkennt man den bereits beschriebenen Thermoelementfühler 24, der der Abtriebswelle 11 der Getriebeeinheit 2 benachbart ist. Er kann also die Temperatur der Schneidwerkzeugeinheit 3 in einem den Schneidkanten relativ nahen Bereich erfassen. Da die Schneidwerkzeugeinheit 3 aus Metall aufgebaut ist, lässt sich damit auch eine hinreichend sichere Aussage über die Temperatur in der Schneidwerkzeugeinheit 3 treffen.

Dieser Thermoelementfühler 24 ist über nicht dargestellte Leitungen und ein Steckverbindungselement 25 sowie ein elektrisches Verbindungselement 26 und ein weiteres Steckverbindungselement 27 sowie eine nicht dargestellte elektrische Steckverbindung zwischen der Getriebeeinheit 2 und der Antriebsmotoreinheit 1 letztlich mit der bereits erwähnten LED-Anzeige 23 bzw. einer dieser zugehörigen Steuerelektronik verbunden. Die Steckverbindungselemente 25 und 27 und die elektrische Verbindung 26 dienen dazu, die Getriebeeinheit 2 zum Nachschmieren oder Reinigen oder zur Reparatur zerlegbar zu gestalten, wie dies in Fig. 4 durch die Darstellung in dem teilweise zerlegten Zustand veranschaulicht ist. Man erkennt dabei insbesondere die verschiedenen Zahnräder einer Getriebeuntersetzung. In dem im Wesentlichen quaderförmigen Gehäuseteil der Getriebeeinheit 2 in der Nachbarschaft der Abtriebswelle 11 kämmen zwei angeschrägte Zahnkränze mit senkrecht zueinander liegenden Rotationsachsen miteinander, von denen einer auf der Abtriebswelle 11 montiert ist. Damit kann die von dem Elektromotor in der Antriebsmotoreinheit 1 erzeugte Drehbewegung über die Getriebeeinheit 2 untersetzt und um 90° in der Rotationsachse verdreht an die Schneidwerkzeugeinheit 3 weitergegeben werden.

Fig. 4 zeigt mit der Bezugsziffer 30 eine Bohrung in einem auskragenden Teil der Getriebeeinheit 2, das unter das Gehäuse der Antriebsmotoreinheit 1 greift. Durch diese Bohrung 30 kann die im Übrigen auf die Abtriebswelle der Antriebsmotoreinheit 1 aufgesteckte Getriebeeinheit 2 an der Antriebsmotoreinheit 1 befestigt werden. Umgekehrt lässt sich die Getriebeeinheit 2 als Modul lediglich durch Lösen der an der Bohrung 30 angebrachten Befestigungsschraube von der Antriebsmotoreinheit 1 abnehmen. Sie bleibt dabei ein zusammenhängender Block aus den in Fig. 4 gezeigten Teilen. Zur Zerlegung entsprechend Fig. 4 müssen also weitere Befestigungsschrauben gelöst werden, die die verschiedenen Teile zusammenhalten. Insbesondere können durch Lösen einiger Befestigungsschrauben die in Fig. 4 der Antriebsmotoreinheit 1 benachbarten beiden Elemente der Getriebeeinheit 2 ( nämlich die Elemente mit den elektrischen Verbindungsteilen 26 und 27) abgenommen und gegen an andere Typen von Antriebsmotoreinheiten 1 angepasste Elemente ausgetauscht werden. Dies muss jedoch vom Benutzer normalerweise nicht durchgeführt werden.

## Patentansprüche

1. Vorrichtung zum Schneiden flacher Gegenstände,
die mit den Händen gehalten werden kann und aufweist:
eine Antriebsmotoreinheit (1) und eine Schneidwerkzeugeinheit (3), die zumindest ein bewegbares und durch die Antriebsmotoreinheit (1) antreibbares Schneidwerkzeug (8, 13) und zumindest ein weiteres Schneidwerkzeug (13, 9) aufweist,
wobei das bewegbare Schneidwerkzeug (8, 13) relativ zu dem weiteren Schneidwerkzeug (13, 9) bewegbar ist und die Bewegung einer Schneidkante des bewegbaren Schneidwerkzeugs (8, 13) einer geschlossenen Bahn entspricht, die eine endliche Fläche umläuft
**dadurch gekennzeichnet, dass** das zumindest eine bewegbare Schneidwerkzeug (8, 13) an einer von seiner Schneidkante entfernten Lagerachse (20, 21) drehbar und verschiebbar gelagert ist und an einem seiner Schneidkante näheren Exzenter (22) gelagert und von diesem antreibbar ist.

2. Vorrichtung nach Anspruch 1, bei der die Schneidwerkzeugeinheit (3) von der Antriebsmotoreinheit (1) abnehmbar ist.

3. Vorrichtung nach Anspruch 1 oder 2 mit einer Getriebeeinheit (2), über die die Antriebsmotoreinheit (1) die Schneidwerkzeugeinheit (3) antreiben kann, wobei die Getriebeeinheit (2) von der Motoreinheit (1) und der Schneidwerkzeugeinheit (3) abnehmbar ist.

4. Vorrichtung nach einem der vorstehenden Ansprüche, bei der das bewegbare Schneidwerkzeug (8, 13) in der Schneidwerkzeugeinheit (3) mit einem Kugellager (22) gelagert ist.

5. Vorrichtung nach einem der vorstehenden Ansprüche mit einer Temperaturerfassungseinrichtung (24) zum Erfassen einer Temperatur in der Schneidwerkzeugeinheit (3).

6. Vorrichtung nach einem der vorstehenden Ansprüche, bei der die Schneidwerkzeuge (8, 9, 13) plan sind und flächig aneinander entlang gleiten.

7. Vorrichtung nach einem der vorstehenden Ansprüche, bei der zumindest das bewegbare Schneidwerkzeug (8, 13) eine konvex gebogene Schneidkante aufweist, die vorzugsweise gezahnt ist.

8. Vorrichtung nach einem der vorstehenden Ansprüche, bei der die Schneidwerkzeugeinheit (3) bei der Verwendung infolge von Reibung zwischen einer Schneidkante des zumindest einen bewegbaren Schneidwerkzeugs (8, 13) und dem zu schneidenden flachen Gegenstand entlang dem flachen Gegenstand transportiert wird.

9. Vorrichtung nach einem der vorstehenden Ansprüche, mit zwei bewegbaren Schneidwerkzeugen (8, 13) und einem zwischen den bewegbaren Schneidwerkzeugen (8, 13) angeordneten feststehenden Schneidwerkzeug (9), wobei die Vorrichtung während des Schneidens mit dem feststehenden Schneidwerkzeug (9) den flachen Gegenstand gleitend untergreift oder gleitend auf ihm abgestützt wird.

10. Vorrichtung nach einem der vorstehenden Ansprüche, bei der die Antriebsmotoreinheit (1) ein stufenlose Geschwindigkeitssteuerung durch den Benutzer gestattet.

11. Vorrichtung zum Schneiden flacher Gegenstände mit einer Schneidwerkzeugeinheit (3) und einer Getriebeeinheit (2), welche Vorrichtung dazu ausgelegt ist, durch Kopplung der Getriebeeinheit (2) mit einer üblichen elektrischen Bohr-/Schraubmaschine (1) als Antriebseinheit (1) eine Vorrichtung gemäß einem der vorstehenden Ansprüche zu bilden.

12. Verfahren zum Schneiden flacher Gegenstände mit einer Vorrichtung nach einem der vorstehenden Ansprüche, bei welchem Verfahren die Vorrichtung in der Hand gehalten und mit der Schneidwerkzeugeinheit (3) an den flachen Gegenstand geführt wird, wobei die Schneidwerkzeugeinheit (3) den flachen Gegenstand durch die Antriebsmotoreinheit (1) angetrieben aufschneidet.

13. Verfahren nach Anspruch 12, bei dem Hartverbände aufgeschnitten werden.

14. Verfahren nach Anspruch 12, bei dem Schutzanzüge, insbesondere Motorradanzüge, aufgeschnitten werden.

## Claims

1. A device for cutting flat objects,
which can be held with the hands and is provided with:
a driving motor unit (1) and a cutting tool unit (3), which at least comprises one cutting tool (8, 13) being movable and drivable by said driving motor unit (1), and at least one further cutting tool (13, 9),
wherein said movable cutting tool (8, 13) is movable relatively to said further cutting tool (13, 9), wherein the movement of a cutting edge of said movable cutting tool (8, 13) corresponds to (belongs to) a closed pathway surrounding a finite area;
wherein said at least one movable cutting tool (8, 13) is held rotatably and displaceably at a bearing axis (20, 21) distant from the cutting edge of said movable cutting tool, and wherein said movable cutting tool is also held at an eccentric (22) located closer to said cutting edge and allowing to drive said movable cutting tool.

2. A device according to claim 1, in which said cutting tool unit (3) is detachable from said driving motor unit (1).

3. A device according to claim1 or 2, comprising a gear unit (2), via which said driving motor unit (1) can actuate said cutting tool unit (3), wherein said gear unit (2) is detachable from said driving motor unit (1) and from said cutting tool unit (3).

4. A device according to one of the preceding claims, in which said movable cutting tool (8, 13) in said cutting tool unit (3) is held by means of a ball bearing (22).

5. A device according one of the preceding claims, comprising a temperature detection device (24) for detecting a temperature in said cutting tool unit (3).

6. A device according to one of the preceding claims, in which said cutting tools (8, 9, 13) are plain and slide along each other with their surfaces.

7. A device according to one of the preceding claims, in which at least said movable cutting tool (8, 13) has a convexly curved cutting edge, which preferably is provided with teeth.

8. A device according to one of the preceding claims, in which said cutting tool unit (3) during operation is transported along said flat object to be cut in consequence of friction between a cutting edge of said at least one movable cutting tool (8, 13) and said flat object.

9. A device according to one of the preceding claims, comprising two movable cutting tools (8, 13) and a static cutting tool (9) being arranged between said movable cutting tools (8, 13), wherein the device during the cutting process slidingly engages with the bottom side of said flat object or is slidingly supported thereupon via its static cutting tool (9).

10. A device according to one of the preceding claims, in which said driving motor unit (1) allows for a continuous regulation of velocity by the operator.

11. A device for cutting flat objects, comprising a cutting tool unit (3) and a gear unit (2), wherein this device is designed to form a device according to one of the preceding claims by coupling said gear unit (2) to a common electrical drilling machine/screwing machine (1) serving as a driving unit (1).

12. A method for cutting flat objects by means of a device according to one of the preceding claims, in which method said device is held with the hand and its cutting tool unit (3) is approached to said flat object, wherein said cutting tool unit (3) cuts open said flat object under the actuation by the driving motor unit (1).

13. A method according to claim 12, in which stiff bandages are cut open.

14. A method according to claim 12, in which protective suits, in particular motorcyclist suits, are cut open.

## Revendications

1. Dispositif pour découper des objets plats, qui peut être tenu manuellement et qui comporte :
une unité de moteur d'entraînement (1) et une unité d'outil de découpe (3), qui comprend au moins un outil de découpe (8, 13) mobile et entraînable par l'unité de moteur d'entraînement (1) et au moins un autre outil de découpe (13, 9),
moyennant quoi l'outil de découpe (8, 13) mobile est mobile par rapport au l'autre outil de découpe (13, 9) et le déplacement d'un bord coupant de l'outil de découpe (8, 13) mobile correspondant à une voie fermée qui entoure une surface terminale,
**caractérisé en ce que** le au moins un outil de découpe (8, 13) mobile est logé de manière à tourner et à coulisser sur un axe de palier (20, 21) éloigné de son bord coupant et **en ce qu'**il est logé sur une excentrique (22) plus proche de son bord coupant et **en ce qu'**il est entraînable par celle-ci.

2. Dispositif selon la revendication 1, dans lequel l'unité d'outil de découpe (3) peut être démontée de l'unité de moteur d'entraînement (1).

3. Dispositif selon la revendication 1 ou 2, avec une unité d'engrenage (2) par l'intermédiaire de laquelle l'unité de moteur d'entraînement (1) peut entraîner l'unité d'outil de découpe (3), moyennant quoi l'unité d'engrenage (2) peut être démontée de l'unité de moteur (1) et de l'unité d'outil de découpe (3).

4. Dispositif selon l'une quelconque des revendications précédentes, dans lequel l'outil de découpe mobile (8, 13) est logée dans l'unité d'outil de découpe (3) avec un roulement à billes (22).

5. Dispositif selon l'une quelconque des revendications précédentes, avec un dispositif de détection de température (24) pour détecter une température dans l'unité d'outil de découpe (3).

6. Dispositif selon l'une quelconque des revendications précédentes, dans lequel les outils de découpe (8, 9, 13) sont plans et glissent à plat le long les uns des autres.

7. Dispositif selon l'une quelconque des revendications précédentes, dans lequel au moins l'outil de découpe mobile (8, 13) présente un bord coupant bombé convexe qui est, de préférence, denté.

8. Dispositif selon l'une quelconque des revendications précédentes, dans lequel l'unité d'outil de découpe mobile (3) est transportée le long de l'objet plat à la suite d'une friction entre un bord coupant du au moins un outil de découpe mobile (8, 13) et l'objet plat à découper.

9. Dispositif selon l'une quelconque des revendications précédentes, avec deux outils de découpe mobiles (8, 13) et un outil de découpe fixe (9) disposé entre les outils de découpe mobiles (8, 13), moyennant quoi le dispositif saisit l'objet plat par-dessous en glissant ou s'appuie sur lui en glissant pendant la découpe avec l'outil de découpe fixe (9).

10. Dispositif selon l'une quelconque des revendications précédentes, dans lequel l'unité de moteur d'entraînement (1) permet une commande de vitesse continue par l'utilisateur.

11. Dispositif pour découper des objets plats avec une unité d'outil de découpe(3) et une unité d'engrenage (2), lequel dispositif est conçu pour former un dispositif selon l'une quelconque des revendications précédentes, par l'accouplement de l'unité d'engrenage (2) avec une perceuse-visseuse électrique courante (1) comme unité d'entraînement (1).

12. Procédé pour découper des objets plats avec un dispositif selon l'une quelconque des revendications précédentes, selon lequel le dispositif est tenu manuellement et guidé avec l'unité d'outil de découpe (3) sur l'objet plat, moyennant quoi l'unité d'outil de découpe (3) découpe l'objet plat entraînée par l'unité de moteur d'entraînement (1).

13. Procédé selon la revendication 12, selon lequel des bandages rigides sont découpés.

14. Procédé selon la revendication 12, selon lequel des vêtements de protection, en particulier des vêtements pour motards, sont découpés.
